# EUROPÄISCHE PATENTSCHRIFT

(11) **EP 4 109 851 B1**
(45) Veröffentlichungstag und Bekanntmachung des Hinweises auf die Patenterteilung: **24.01.2024**
(21) Anmeldenummer: 22173588.9
(22) Anmeldetag: 16.05.2022
(51) Int. Cl.: H04L 49/00, H04L 47/56, A61B 6/03

(54) **DATENÜBERTRAGUNGSEINRICHTUNG, MEDIZINISCHE BILDGEBUNGSEINRICHTUNG UND VERFAHREN ZUR ÜBERTRAGUNG VON DATENPAKETEN**
DATA TRANSMISSION DEVICE, MEDICAL IMAGING DEVICE AND DATA PACKET TRANSMISSION METHOD
DISPOSITIF DE TRANSMISSION DE DONNÉES, DISPOSITIF D'IMAGERIE MÉDICALE ET PROCÉDÉ DE TRANSMISSION DE PAQUETS DE DONNÉES

(30) Priorität: 23.06.2021 DE 102021206498
(43) Veröffentlichungstag der Anmeldung: 28.12.2022
(73) Patentinhaber: Siemens Healthcare GmbH, 91052 Erlangen (DE)
(72) Erfinder: Karl, Harald, 90765 Fürth (DE); Kiesel, Jutta, 91301 Forchheim (DE); LIU, BING, Shanghai, 201318 (CN); Urban, Andreas, 90542 Eckental (DE)
(74) Vertreter: Siemens Healthineers Patent Attorneys

(56) Entgegenhaltungen:
- EP-A1- 2 797 269
- DE-A1-102012 201 222
- US-A1- 2017 034 843
- US-A1- 2019 334 837

## Beschreibung

Die Erfindung betrifft eine Datenübertragungseinrichtung zur Übertragung von Datenpaketen, insbesondere von Messdaten einer medizinischen Bildgebungseinrichtung, umfassend wenigstens eine Empfangsschnittstelle zum Empfangen von Datenpaketen von einer jeweiligen Datenquelle, insbesondere über eine drahtgebundene Verbindung, einen jeweiligen Empfangspuffer zur Zwischenspeicherung der über die jeweilige Empfangsschnittstelle empfangenen Datenpakete, eine Transfereinrichtung zur Übertragung der Datenpakete von dem jeweiligen Empfangspuffer in einen für das jeweilige Datenpaket aus mehreren vorhandenen Sendepuffern ausgewählten Sendepuffer, und eine jeweilige Sendeschnittstelle zum Senden der in dem jeweiligen Sendepuffer gespeicherten Datenpakete an eine Empfangseinrichtung, insbesondere über eine drahtlose Verbindung. Daneben betrifft die Erfindung eine medizinische Bildgebungseinrichtung und ein Verfahren zur Übertragung von Datenpaketen.

Im Bereich der medizinischen Bildgebung, beispielsweise in Computertomographen, und auch in anderen Anwendungsgebieten kann es erforderlich sein, Daten zwischen relativ zueinander bewegten Komponenten zu übertragen. So sind beispielsweise die bildgebenden Röntgensensoren bei Computertomographen typischerweise an einer bezüglich einer Basis drehbar gelagerten Gantry angeordnet und die Datenverarbeitung beziehungsweise Visualisierung soll durch bezüglich der Basis feststehende Komponenten, beispielsweise Arbeitsplatzrechner oder feststehende Bildprozessoren, erfolgen. Hierbei können beispielsweise bei modernen Computertomographen sehr hohe Datenraten von beispielsweise 35 Gbit/s verwendet werden, wodurch hohe Anforderungen an eine solche Kommunikationsverbindung resultieren.

Prinzipiell kann eine solche Datenübertragung zwar beispielsweise über Schleifkontakte erfolgen. Um hohe Datenraten bei geringem Wartungsaufwand und geringer Störanfälligkeit zu erreichen, kann es jedoch vorteilhaft sein, stattdessen eine drahtlose Datenübertragung über kurze Strecken zu nutzen. Die Datenübertragung kann hierbei beispielsweise per Funk beziehungsweise allgemein durch Hochfrequenztechnik oder über eine kapazitive Kopplung erfolgen.

Hierbei ist es aus mehreren Gründen vorteilhaft, mehrere gleichzeitig nutzbare Funkkanäle vorzusehen. Zum einen kann hierdurch die Datenübertragungsrate erhöht werden. Zum anderen werden, beispielsweise aufgrund der Rotation einer Gantry, genutzte Sendeantennen an genutzten Empfangsantennen vorbeigeführt, so dass sich beispielsweise einzelne Sendeantennen vorübergehend in Bereichen befinden können, in denen ihre Signale nicht robust durch Empfangsantennen empfangen werden können. Es ist daher vorteilhaft, mehrere Sende- und Empfangsantennen zu nutzen, so dass beispielsweise unabhängig vom Drehwinkel einer Gantry stets eine Kommunikation über zumindest einen oder zumindest eine bestimmte Anzahl von Kanälen möglich ist.

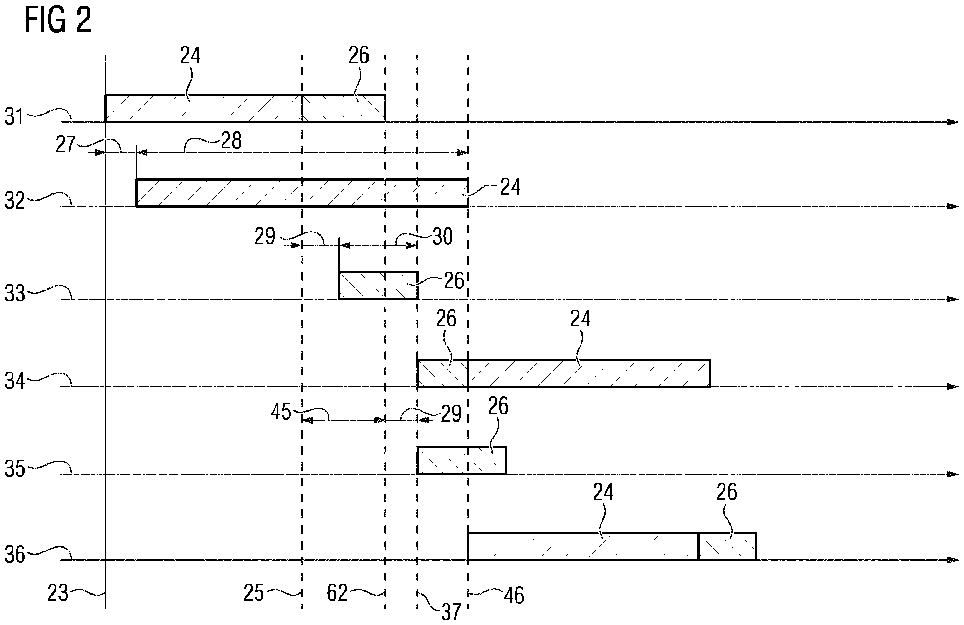

Da bei hybriden Übertragungssystemen, bei denen ein Teil des Übertragungspfades kabelgebunden und ein Teil des Übertragungspfades drahtlos ist, in den verschiedenen Teilen des Übertragungspfades typischerweise unterschiedlich hohe Übertragungsraten erreicht werden, werden im Bereich der Übergänge zwischen einer drahtgebundenen und einer drahtlosen Übertragung und umgekehrt typischerweise "store and forward"-Ansätze, also insbesondere eine FIFO-Pufferung (First in, First out) von Datenpaketen, genutzt. Wie später noch mit Bezug auf Fig. 2 an einem Beispiel erläutert werden wird, kann die Kombination aus der Nutzung mehrerer Übertragungskanäle und der genutzten Pufferung in einigen Fällen dazu führen, dass sich die Reihenfolge von übertragenen Datenpaketen ändert.

Dies könnte zwar prinzipiell in Kauf genommen werden, beispielsweise indem eine die Datenpakete verarbeitende Verarbeitungseinrichtung, beispielsweise ein Arbeitsplatzrechner, der im Rahmen einer Computertomographie zur Generierung der dreidimensionalen Bilddaten genutzt wird, die empfangenen Pakete zunächst speichert und in die richtige Reihenfolge bringt, bevor eine Weiterverarbeitung erfolgt. Da jedoch beispielsweise bei einer Computertomographie sehr hohe Datenraten genutzt werden, würde eine solche nachträgliche Umordnung empfangener Datenpakete, insbesondere wenn eine Visualisierung von Messdaten nahezu in Echtzeit erfolgen soll, zu erheblichen zusätzlichen Anforderungen bezüglich der benötigten Rechenzeit und der Schnelligkeit und Menge des vorhandenen Speichers führen. Daher wäre beispielsweise eine echtzeitnahe Datenvisualisierung zumindest bei Geräten im unteren Preissegment nur mit erheblichen Einschränkungen umsetzbar.

Die Patentschrift US2019334837 A1 legt ein Verfahren zur Verwaltung von Sendebuffern in einem Netzwerkswitch offen. 1

Die Patentschrift US 2017/034843 A1 legt ein Verfahren offen, bei dem unterschiedliche Netzwerklatenzen durch Scheduling ausgeglichen werden.

Der Erfindung liegt somit die Aufgabe zugrunde, eine demgegenüber verbesserte Möglichkeit anzugeben, auch bei Kombination einer Datenpufferung mit mehreren Übertragungskanälen eine korrekte Paketreihenfolge zu erreichen.

Diese Aufgabe wird erfindungsgemäß durch eine Datenübertragungseinrichtung der eingangs genannten Art gelöst, wobei die Transfereinrichtung dazu eingerichtet ist, die Übertragung des jeweiligen Datenpakets von dem jeweiligen Empfangspuffer in den ausgewählten Sendepuffer erst dann durchzuführen, wenn eine Freigabebedingung erfüllt ist, deren Erfüllung von einem Füllgrad eines von dem ausgewählten Sendepuffer unterschiedlichen weiteren der Sendepuffer abhängt.

Die erfindungsgemäße Verzögerung der Übertragung des Datenpakets in den ausgewählten Sendepuffer bis die Freigabebedingung erfüllt ist, ermöglicht es, eine Änderung der Paketreihenfolge durch die parallele Datenübertragung über die den verschiedenen Sendepuffern zugeordneten Sendeschnittstellen unmittelbar durch eine geeignete Verzögerung zu verhindern, so dass die Pakete durch die Empfangseinrichtung robust in der richtigen Reihenfolge empfangen werden und somit keine nachträgliche Korrektur der Paketreihenfolge erforderlich ist. Dies ermöglicht die Weiterverarbeitung der empfangenen Datenpakete mit erheblich geringerem technischem Aufwand. Zugleich wurde erkannt, dass die Verzögerung einzelner Datenpakete, wenn die Freigabebedingung nicht erfüllt ist, in realen Anwendungsfällen die erreichbare Datenrate nur unwesentlich reduziert und auch durch die Datenübertragung resultierende Latenzen kaum erhöht. Somit können beispielsweise die Vorteile einer parallelen drahtlosen Datenübertragung genutzt werden, ohne dass eine aufwendige Nachverarbeitung der Daten zur Korrektur der Paketreihenfolge erforderlich wird.

Die Transfereinrichtung kann insbesondere jeweils das älteste Datenpaket, also beispielsweise die älteste Nachricht, aus dem Empfangspuffer in den jeweils ausgewählten Sendepuffer übertragen. Der Empfangspuffer kann somit einen FIFO-Puffer (First in, First out) implementieren. Der Sendepuffer kann ebenfalls als FIFO-Puffer für die Sendeschnittstelle dienen, das heißt die Sendeschnittstelle kann zuerst jene Daten aus dem Sendepuffer auslesen, die am frühesten in diesen Puffer geschrieben wurden.

Der ausgewählte Sendepuffer kann in Abhängigkeit des Betriebszustands der Datenübertragungseinrichtung oder einer größeren Einrichtung, die diese umfasst, beispielsweise einer medizinischen Bildgebungseinrichtung, gewählt werden. Wie bereits erläutert, kann es beispielsweise möglich sein, dass bestimmte Sendeschnittstellen, z.B. aufgrund der aktuellen Position ihrer Sendeantennen, vorübergehend Datenpakete nicht an die Empfangseinrichtung übertragen können. Es kann daher zweckmäßig sein, die genutzte Sendeschnittstelle und somit den Sendepuffer beispielsweise in Abhängigkeit einer Rotationsstellung einer Gantry zu wählen. Zudem kann, um eine optimale Ausnutzung der vorhandenen Übertragungskanäle und somit der verfügbaren Bandbreite zu erreichen, ein leerer oder möglichst leerer Sendepuffer ausgewählt werden.

Die Transfereinrichtung kann dazu eingerichtet sein, im Rahmen der Auswertung der Freigabebedingung jenen der Sendepuffer als weiteren Sendepuffer zu berücksichtigen, in den ein weiteres Datenpaket übertragen wurde, das unmittelbar vor dem in den ausgewählten Sendepuffer zu übertragenden Datenpaket empfangen wurde.

Insbesondere kann die Transfereinrichtung dazu eingerichtet sein, im Rahmen der Auswertung der Freigabebedingung jenen der Sendepuffer als weiteren Sendepuffer zu berücksichtigen, in den ein weiteres Datenpaket übertragen wurde, das unmittelbar vor dem in den ausgewählten Sendepuffer zu übertragenden Datenpaket über die gleiche Empfangsschnittstelle empfangen wurde.

In den meisten Anwendungsfällen, insbesondere wenn die Datenübertragungsrate bezüglich des Empfangs durch die Empfangsschnittstelle und des Sendens durch die Sendeschnittstelle nicht allzu stark voneinander abweichen, ist bereits unabhängig von der Prüfung der Freigabebedingung systemimmanent, dass die Übertragung des vorletzten Datenpakets vor der Übertragung des aktuellen Datenpakets abgeschlossen wird. Es ist daher in der Regel ausreichend, im Rahmen der Freigabebedingung sicherzustellen, dass die Übertragung des unmittelbar vorangehenden Datenpakets zur Empfangseinrichtung beziehungsweise der Empfangseinrichtung nachgeschalteten weiteren Einrichtungen, beispielsweise einer Verarbeitungseinrichtung, vor der Übertragung des aktuellen Datenpakets dorthin abgeschlossen ist.

Die Erfüllung der Freigabebedingung kann zusätzlich von der Länge des in den ausgewählten Sendepuffer zu übertragenden Datenpakets abhängen. Die Länge des Datenpakets kann beispielsweise in Bytes oder allgemein in Zeichen angegeben werden. Es ist möglich, dass die Länge des Datenpakets beim Empfangen beziehungsweise beim Schreiben in den Empfangspuffer ermittelt wird. Bei einer Vielzahl von üblichen Kommunikationsprotokollen umfassen die Datenpakete selbst Informationen zu ihrer Länge, beispielsweise in einem Header. Die Länge des Datenpakets kann auch anhand von spezifischen Anfangs- und Endmarkierungen ermittelt werden.

Wie im Folgenden noch genauer erläutert werden wird, kann eine Verzögerung zur Sicherstellung der korrekten Paketreihenfolge insbesondere für relativ kurze Datenpakete relevant sein, weshalb eine Längenberücksichtigung vorteilhaft ist.

Der Füllgrad soll gemäß der unabhängigen Ansprüche, eine Länge eines noch nicht über die dem weiteren Sendepuffer zugeordnete Sendeschnittstelle gesendeten, verbleibenden Teildatenpakets des oder eines vorangehend in den weiteren Sendepuffer übertragenen weiteren Datenpakets beschreiben, wobei die Freigabebedingung erfüllt wird oder nur dann erfüllbar ist, wenn die Länge des in den ausgewählten Sendepuffer zu übertragenden Datenpakets die Länge des Teildatenpakets oder die Summe aus der Länge des Teildatenpakets und einem vorgegebenen Offsetwert erreicht oder überschreitet.

Eine unerwünschte Veränderung der Paketreihenfolge würde dann resultieren, wenn die Übertragung eines später gesendeten Datenpakets zu der Empfangseinrichtung oder einer der Empfangseinrichtung nachgelagerten Einrichtung vollständig abgeschlossen wäre, bevor die Übertragung eines früher gesendeten Datenpakets dorthin abgeschlossen ist, da Datenpakete in der Regel erste nach vollständigem Empfang weitergeleitet beziehungsweise weiterverarbeitet werden. In der Regel, insbesondere dann, wenn die Übertragung der Datenpakete von der Sendeschnittstelle zu der Empfangseinrichtung drahtlos erfolgt, resultieren für die Übertragung aus dem Sendepuffer zu der Empfangseinrichtung beziehungsweise zu dieser nachgeschalteten Einrichtung niedrigere Übertragungsgeschwindigkeiten als sie für eine Übertragung von dem Empfangspuffer in den Sendepuffer erreicht werden. Ist die Länge des verbleibenden Teildatenpakets somit größer als die Länge des in den Sendepuffer zu übertragenden Teildatenpakets, kann dies dazu führen, dass die erforderliche Zeit zur vollständigen Übertragung des Teildatenpakets zur Empfangseinrichtung beziehungsweise einer dieser nachgelagerten Einrichtung die Summe aus der relativ kurzen erforderlichen Zeit zur Übertragung des Datenpakets in dem Sendepuffer und der Zeit zur Übertragung dieses Datenpakets aus dem Sendepuffer zu der Empfangseinrichtung beziehungsweise der nachgelagerten Einrichtung überschreitet, was zu einer Änderung der Reihenfolge der Datenpakete führen würde. Durch das beschriebene Vorgehen kann dies vermieden werden.

In vielen Anwendungsfällen können Übertragungsraten beziehungsweise Laufzeiten für den Übertragungsweg von dem Sendepuffer zu der Empfangseinrichtung beziehungsweise zu nachgelagerten Einrichtungen in gewissem Rahmen variieren. Beispielsweise kann die erforderliche Übertragungszeit für eine drahtlose Übertragung von der Relativposition von Sender und Empfänger abhängen, die sich beispielsweise bei einer Übertragung von einer bewegten Gantry zu einer feststehenden Basis in Abhängigkeit der Drehstellung der Gantry ändern kann. Zudem können Übertragungsstörungen zu einer Abweichung dieser Zeit führen. Auch bei kabelgebundenen Übertragungen können, beispielsweise aufgrund von mehreren möglichen Übertragungspfaden und/oder einer vorübergehenden Pufferung im Rahmen der Übertragung, leichte Variationen der Übertragungszeit resultieren.

Die variable Übertragungszeit kann dazu führen, dass selbst in Fällen, in denen das in den Sendepuffer zu übertragende Datenpaket etwas länger ist als das verbleibende Teildatenpaket, dieses Datenpaket durch die Empfangseinrichtung beziehungsweise eine nachgelagerte Einrichtung vollständig empfangen wird, bevor das verbleibende Teildatenpaket vollständig empfangen wird, also dass sich die Reihenfolge der Datenpakete ändert. Dieses Problem kann durch Nutzung des vorgegebenen Offsets kompensiert werden. Ein geeigneter Offset kann beispielsweise bereits im Rahmen des Systemdesignes abgeschätzt beziehungsweise durch Testmessungen ermittelt und anschließend fest vorgegeben werden.

Die Länge des verbleibenden Teildatenpakets ist in der Regel ohnehin bekannt, da einerseits die Länge des Datenpakets und andererseits die Menge der aus diesem bereits übertragenden Daten bekannt sind. Alternativ wäre es möglich, die Länge des Teildatenpakets beziehungsweise auch eines in einem Puffer gelegten Datenpakets beispielsweise dadurch zu ermitteln, dass ein Abstand zwischen einem Lesezeiger und einem Schreibzeiger auf dem entsprechenden Puffer ermittelt wird oder es können komplexere Datenstrukturen genutzt werden, bei denen Einträge in eine Datenstruktur gezählt werden, beispielsweise im Rahmen einer Objektorientierung.

Die jeweilige Empfangsschnittstelle kann drahtgebunden mit der jeweiligen Datenquelle verbunden oder verbindbar sein. Ergänzend oder alternativ kann die jeweilige Sendeschnittstelle zur drahtlosen Übertragung des jeweiligen Datenpakets an die Empfangseinrichtung ausgebildet sein. Es kann somit insbesondere ein hybrider Übertragungsweg genutzt werden, bei dem in Abschnitten des Übertragungsweges eine drahtgebundene und in anderen Abschnitten des Übertragungsweges eine drahtlose Übermittlung der Datenpakete erfolgt. Dennoch kann, wie obig erläutert, sichergestellt werden, dass die Datenpakete in der richtigen Reihenfolge beim Empfänger ankommen.

Wie bereits erläutert, kann die Datenübertragungseinrichtung insbesondere zur Übertragung von Messdaten einer medizinischen Bildgebungseinrichtung als Datenpakete dienen.

Neben der erfindungsgemäßen Datenübertragungseinrichtung betrifft die Erfindung eine medizinische Bildgebungseinrichtung, insbesondere einen Computertomograph, wobei die Bildgebungseinrichtung eine erfindungsgemäße Datenübertragungseinrichtung umfasst. Wie obig detailliert dargestellt, kann die erfindungsgemäße Datenübertragungseinrichtung besonders vorteilhaft sein, wenn Daten mit relativ hohen Datenraten zwischen relativ zueinander bewegten Komponenten übertragen werden sollen, wie es beispielsweise in Computertomographen und anderen medizinischen Bildgebungseinrichtungen der Fall ist.

Die Bildgebungseinrichtung kann eine Sensoreinrichtung umfassen, die insbesondere ein Röntgensensor ist oder einen Röntgensensor umfasst, wobei die Sensoreinrichtung dazu eingerichtet ist, als die Datenquelle oder über eine als Datenquelle dienende Verarbeitungseinrichtung Datenpakete an die Datenübertragungseinrichtung bereitzustellen. Insbesondere kann die Sensoreinrichtung Bilddaten bereitstellen, beispielsweise Röntgenbilder.

Die medizinische Bildgebungseinrichtung kann ein bezüglich einer Basis beweglich gelagertes Gestell, insbesondere eine drehbar gelagerte Gantry, umfassen, das die Datenquelle und die Datenübertragungseinrichtung umfasst, wobei die Empfangseinrichtung ortsfest bezüglich der Basis angeordnet ist. Hierbei kann die Datenübertragungseinrichtung insbesondere zur Übertragung von einem bewegten Koordinatensystem, beispielsweise einer rotierenden Gantry, in ein stehendes Koordinatensystem dienen. Wird eine kurzreichweitige drahtlose Übertragung genutzt, können je nach Bewegungsposition beziehungsweise Rotationswinkel des Gestells bezüglich der Basis jeweils nur Teile der Sendeschnittstellen bzw. der diesen zugeordneten Sendeantennen derart angeordnet sein, dass ihr Signal robust durch eine jeweilige Empfangsantenne der Empfangseinrichtung empfangen werden kann. Dies kann bei der Auswahl des Sendepuffers, in den ein Datenpaket übertragen wird, und somit der zu nutzenden Sendeschnittstelle berücksichtigt werden.

Wie bereits erwähnt, kann die Bildgebungseinrichtung ein Computertomograph sein oder einen Computertomograph umfassen, und/oder die Sensoreinrichtung kann ein Röntgensensor sein oder einen Röntgensensor umfassen, und/oder das beweglich gelagerte Gestell kann eine drehbar gelagerte Gantry sein.

Die Erfindung betrifft zudem ein Verfahren zur Übertragung von Datenpaketen, wobei
- Datenpakete von einer jeweiligen Datenquelle empfangen und in einem Empfangspuffer zwischengespeichert werden,
- die Datenpakete von dem Empfangspuffer in einen für das jeweilige Datenpaket aus mehreren vorhandenen Sendepuffern ausgewählten Sendepuffer übertragen werden, wobei die Übertragung des jeweiligen Datenpakets von dem jeweiligen Empfangspuffer in den ausgewählten Sendepuffer erst bei Erfüllung einer Freigabebedingung erfolgt, deren Erfüllung von einem Füllgrad eines von dem ausgewählten Sendepuffer unterschiedlichen weiteren der Sendepuffer abhängt, und
- die in dem jeweiligen Sendepuffer gespeicherten Datenpakete durch eine jeweilige dem Sendepuffer zugeordnete Sendeschnittstelle an eine Empfangseinrichtung gesendet werden.

Insbesondere können in dem Verfahren als Datenpakete Messdaten einer medizinischen Bildgebungseinrichtung übertragen werden. Ergänzend oder alternativ können die Datenpakete von der jeweiligen Datenquelle über eine drahtgebundene Verbindung empfangen werden. Ergänzend oder alternativ können die in dem jeweiligen Sendepuffer gespeicherten Datenpakete durch die jeweilige dem Sendepuffer zugeordnete Sendeschnittstelle über eine drahtlose Verbindung an die Empfangseinrichtung gesendet werden.

Das erfindungsgemäße Verfahren kann insbesondere durch die erfindungsgemäße Datenübertragungseinrichtung implementiert werden beziehungsweise in der erfindungsgemäßen medizinischen Bildgebungseinrichtung zur Datenübertragung genutzt werden. Insbesondere kann die erfindungsgemäße Datenübertragungseinrichtung zur Durchführung des erfindungsgemäßen Verfahrens eingerichtet sein. Unabhängig hiervon lassen sich die zur erfindungsgemäßen Datenübertragungseinrichtung beziehungsweise zur erfindungsgemäßen medizinischen Bildgebungseinrichtung erläuterten Merkmale mit den dort genannten Vorteilen auf das erfindungsgemäße Verfahren übertragen und umgekehrt.

Insbesondere kann im Rahmen der Auswertung der Freigabebedingung jener der Sendepuffer als weiterer Sendepuffer berücksichtigt werden, in den ein weiteres Datenpaket übertragen wurde, das unmittelbar vor dem in den ausgewählten Sendepuffer zu übertragenden Datenpaket empfangen wurde. Ergänzend oder alternativ kann die Erfüllung der Freigabebedingung zusätzlich von der Länge des in den ausgewählten Sendepuffers zu übertragenden Datenpakets abhängen. Für diesbezügliche Weiterbildungen wird auf die obigen Erläuterungen zur erfindungsgemäßen Datenübertragungseinrichtung verwiesen.

Die Erfindung betrifft zudem ein Computerprogram, das Anweisungen umfasst, bei deren Ausführung durch eine Transfereinrichtung Datenpakete von einem Empfangspuffer in einen für das jeweilige Datenpaket aus mehreren vorhandenen Sendepuffern ausgewählten Sendepuffer übertragen werden, wobei die Übertragung des jeweiligen Datenpakets von dem jeweiligen Empfangspuffer in den ausgewählten Sendepuffer erst bei Erfüllung einer Freigabebedingung erfolgt, deren Erfüllung von einem Füllgrad eines von dem ausgewählten Sendepuffer unterschiedlichen weiteren der Sendepuffer abhängt. Als Transfereinrichtung kann jegliche programmierbare Verarbeitungseinrichtung dienen, die zumindest lesend auf einen Empfangspuffer bzw. einen diesen bildenden Speicherbereich und zumindest schreibend auf mehrere Sendepuffer bzw. jeweilige die Sendepuffer bildende Speicherbereiche zugreifen kann. Als solche Verarbeitungseinrichtung bzw. Transfereinrichtung kann z.B. eine CPU, ein Microcontroller oder ein FPGA dienen.

Die Anweisungen des Computerprogramms können insbesondere jenen Teil des erfindungsgemäßen Verfahrens implementieren, in dem die Datenpakete von dem Empfangspuffer in den ausgewählten Sendepuffer übertragen werden und in dem die Freigabebedingung geprüft wird. Obig zum erfindungsgemäßen Verfahren erläuterte Merkmale können somit entsprechend auf das erfindungsgemäße Computerprogramm übertragen werden.

Die durch das erfindungsgemäße Computerprogramm programmierte Transfereinrichtung kann als Transfereinrichtung in der erfindungsgemäßen Datenübertragungseinrichtung bzw. der erfindungsgemäßen Bildgebungseinrichtung verwendet werden. Zu diesen Gegenständen offenbarte Merkmale können daher mit den genannten Vorteilen auf das erfindungsgemäße Computerprogramm übertragen werden.

Die Erfindung betrifft zudem einen computerlesbaren Datenträger, der ein erfindungsgemäßes Computerprogramm umfasst.

Die beschriebene Funktionalität bzw. die in der erfindungsgemäßen Datenübertragungseinrichtung genutzte Transfereinrichtung kann statt durch ein Computerprogramm auch durch Festverdrahtung einer entsprechenden Funktionalität, z.B. durch einen ASIC, implementiert werden.

Weitere Vorteile und Einzelheiten der Erfindung ergeben sich aus den folgenden Ausführungsbeispielen sowie den zugehörigen Zeichnungen. Hierbei zeigen schematisch:
- Fig. 1: ein Ausführungsbeispiel einer erfindungsgemäßen medizinischen Bildgebungseinrichtung, die ein Ausführungsbeispiel der erfindungsgemäßen Datenübertragungseinrichtung umfasst, wobei zur Übertragung der Datenpakete ein Ausführungsbeispiel des erfindungsgemäßen Verfahrens genutzt wird,
- Fig. 2: die Auswirkung der Auswertung in der Freigabebedingung auf den zeitlichen Ablauf der Übertragung von Datenpaketen in der in Fig. 1 gezeigten medizinischen Bildgebungseinrichtung,
- Fig. 3: eine Illustration zur Verdeutlichung der Auswertung der Freigabebedingung, und
- Fig. 4: ein weiteres Ausführungsbeispiel einer erfindungsgemäßen medizinischen Bildgebungseinrichtung.

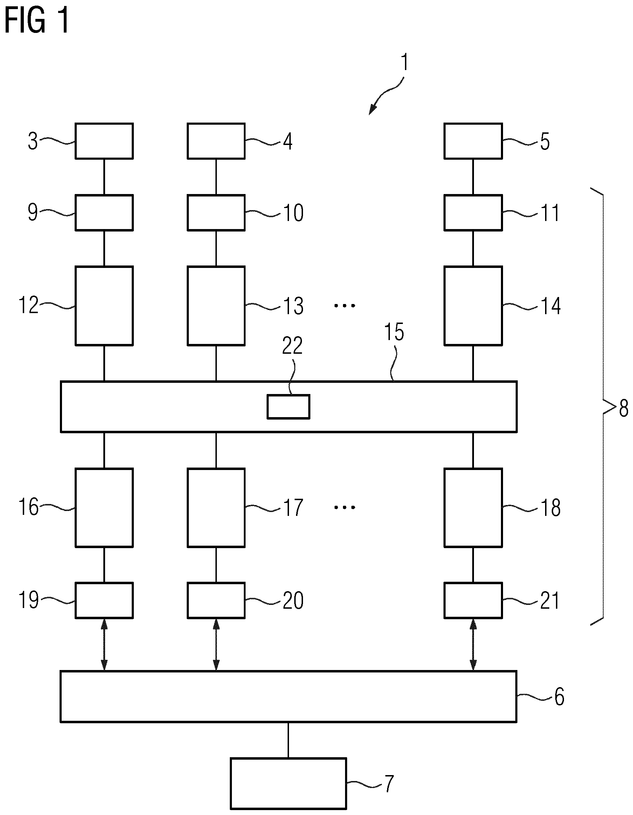

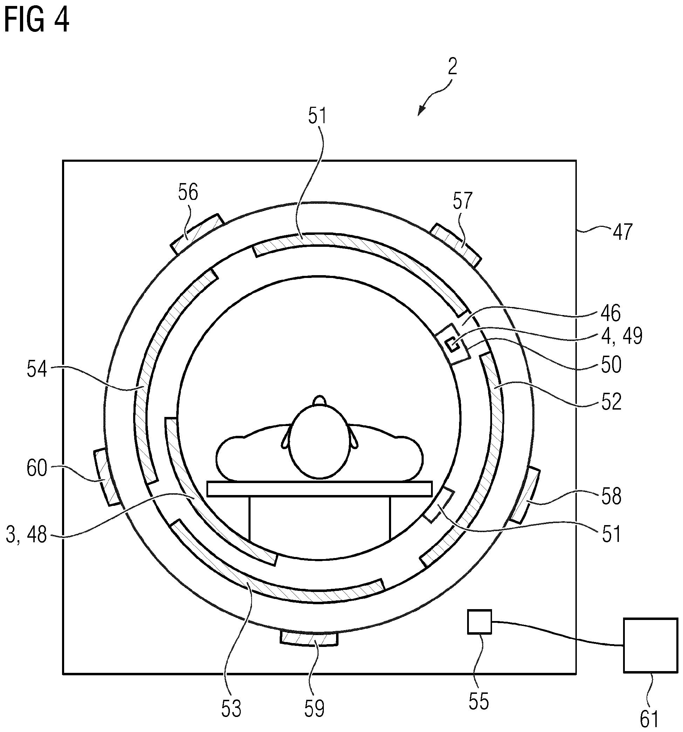

Fig. 1 zeigt schematisch Komponenten einer medizinischen Bildgebungseinrichtung, die bezüglich der Bereitstellung, Übertragung und Verarbeitung von Messdaten relevant sind. Eine konkrete Ausgestaltung einer entsprechenden medizinischen Bildgebungseinrichtung als Computertomograph wird später noch mit Bezug auf Fig. 4 genauer erläutert werden.

Die Bildgebungseinrichtung 1 umfasst mehrere Datenquellen 3, 4, 5, die insbesondere durch Sensoreinrichtungen, beispielsweise durch einen Röntgensensor eines Computertomographen, gebildet sind. Alternativ können die Datenquellen 3, 4, 5 zumindest teilweise auch durch Verarbeitungseinrichtungen gebildet sein, die bereits eine Vorverarbeitung von durch Sensoren bereitgestellte Messdaten durchführen. Die bereitgestellten Messdaten beziehungsweise diese umfassende Datenpakete sollen über eine Empfangseinrichtung 6 an eine Verarbeitungseinrichtung 7 bereitgestellt werden.

Beispielsweise bei Computertomographen ist es hierbei üblich, dass die Datenquellen bezüglich einer Basis und somit typischerweise auch bezüglich der Verarbeitungseinrichtung 7 im Rahmen der Messung bewegt werden, beispielsweise weil sie auf einer rotierenden Gantry angeordnet sind. Der Übergang von dem bewegten Bezugssystem der rotierenden Gantry auf das stehende Bezugssystem der Basis wird im Beispiel durch Nutzung einer Datenübertragungseinrichtung 8 ermöglicht, an die die Datenpakete durch die Datenquellen 3, 4, 5 drahtgebunden übertragen werden und die beispielsweise ebenfalls an der Gantry angebracht sein kann. Die Übertragung von der Datenübertragungseinrichtung 8 an die Empfangseinrichtung 6 erfolgt hingegen drahtlos, so dass auf die Nutzung von Schleifkontakten oder ähnlichem zur Datenübertragung verzichtet werden kann.

Die Datenübertragungseinrichtung 8 umfasst mehrere Empfangsschnittstellen 9, 10, 11, über die jeweils Datenpakete einer der Datenquellen 3, 4, 5 kabelgebunden empfangen werden. Die empfangenen Datenpakete werden zunächst in einem jeweiligen Empfangspuffer 12, 13, 14 abgelegt und von dort durch eine Transfereinrichtung 15 in einem ausgewählten von mehreren Sendepuffern 16, 17 18 abgelegt. Verschiedene Sendeschnittstellen 19, 20, 21 übertragen jeweils die in einem der Sendepuffer 16, 17, 18 abgelegten Datenpakete.

Die Nutzung mehrerer Sendeschnittstellen 19, 20, 21 und zugeordneter Sendepuffer 16, 17, 18 ist einerseits zweckmäßig, wenn nicht zu jedem Betriebszeitpunkt, also beispielsweise nicht in jeder Drehstellung einer Gantry, jede der Sendeschnittstellen 19, 20, 21 genutzt werden kann, um Datenpakete zu senden, beispielsweise weil eine genutzte Sendeantenne nicht im Bereich einer Empfangsantenne der Empfangseinrichtung 6 angeordnet ist. Zudem kann durch Verteilung der empfangenen Datenpakete auf unterschiedliche Sendeschnittstellen 19, 20, 21 eine Parallelisierung der Datenübertragung und somit ein höherer Datendurchsatz erreicht werden.

Im gezeigten Beispiel werden empfangene Datenpakete von der Empfangseinrichtung 6 nur an eine einzige weitere Einrichtung, nämliche eine Verarbeitungseinrichtung 7, bereitgestellt. Es wäre jedoch auch möglich, empfangene Datenpakete an mehrere Einrichtungen bereitzustellen beziehungsweise eine Adressinformation in den Datenpaketen zu nutzen, so dass die Empfangseinrichtung 6 oder eine nachgelagerte Einrichtung die Datenpakete in Abhängigkeit ihres Inhalts an verschiedene weitere Einrichtungen verteilen kann.

Zum Erreichen von minimalen Verzögerungen bei der Datenübertragung und eines maximalen Datendurchsatzes wäre es prinzipiell ideal, wenn die Transfereinrichtung 15 in einem der Empfangspuffer 12, 13, 14 vorhandene Daten unmittelbar und verzögerungsfrei in den Sendepuffer 16, 17, 18 jener Sendeschnittstelle 19, 20, 21 schreiben würde, durch die eine Übertragung dieser Daten erfolgen soll. Durch die Nutzung von parallelen Übertragungspfaden für die drahtlose Datenübertragung, unterschiedlichen Übertragungsraten, die für die drahtgebundene und drahtlose Datenübertragung erreicht werden, und die Nutzung unterschiedlicher Paketlängen von Datenpaketen können bei einem solchen vorgehen jedoch Situationen auftreten, bei denen Datenpakete durch die Empfangseinrichtung 6 beziehungsweise die Verarbeitungseinrichtung 7 in einer unterschiedlichen Reihenfolge empfangen werden, als sie durch die jeweilige Datenquelle 3, 4, 5 gesendet wurden. Ein Beispiel für eine solche Situation wird im Folgenden noch mit Bezug auf Fig. 2 erläutert werden.

Prinzipiell wäre es zwar möglich, eine falsche Reihenfolge der empfangenen Datenpakete durch ein nachträgliches Umsortieren zu korrigieren. Da jedoch beispielsweise im Bereich der Computertomographie sehr hohe Datenraten bei der Übertragung der Messergebnisse von beispielsweise 35 Gbit/s genutzt werden, ist ein nachträgliches Umsortieren der empfangenen Pakete technisch relativ aufwendig.

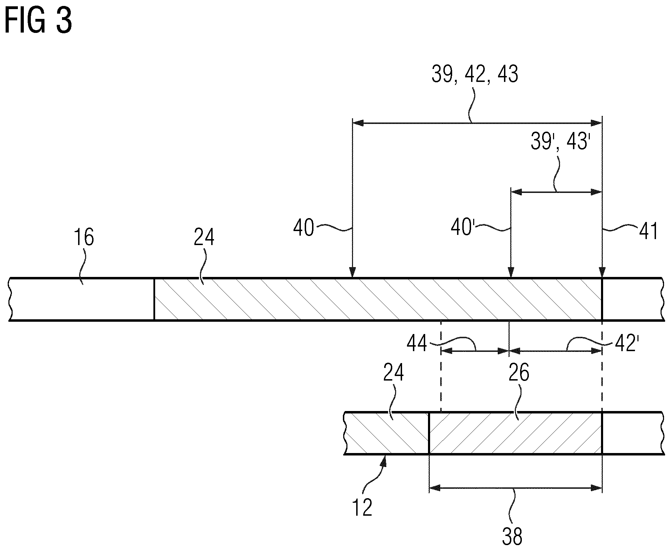

In der medizinischen Bildgebungseinrichtung 1 beziehungsweise in der Datenübertragungseinrichtung 8 wird daher bereits im Rahmen der Datenübertragung verhindert, dass eine solche Fehlordnung der Datenpakete an der Empfangseinrichtung 6 beziehungsweise der Verarbeitungseinrichtung 7 resultiert. Um dies zu ermöglichen, ist die Transfereinrichtung 15 dazu eingerichtet, eine Übertragung eines jeweiligen Datenpakets von dem jeweiligen Empfangspuffer 12, 13, 14 in den ausgewählten Sendepuffer 16, 17, 18 erste dann durchzuführen, wenn eine Freigabebedingung 22 erfüllt ist, deren Erfüllung von einem Füllgrad eines von dem ausgewählten Sendepuffer 16, 17, 18 unterschiedlichen weiteren der Sendepuffer 16, 17, 18 abhängt. Die Wirkung und Implementierung dieser Freigabebedingung 22 werden im Folgenden mit Bezug auf die Figuren 2 und 3 näher erläutert.

Fig. 2 zeigt in mehreren Zeilen 31 - 36 jeweils den zeitlichen Verlauf unterschiedlicher Übertragungsvorgänge in der medizinischen Bildgebungseinrichtung 1. Zunächst sollen die Übertragungsvorgänge für den Fall erläutert werden, dass in einem Empfangspuffer 12, 13, 14 abgelegte Daten unmittelbar in einen jeweiligen ausgewählten Sendepuffer 16, 17, 18 übertragen werden. Dies entspricht dem Fall, dass die Freigabebedingung 22 nicht ausgewertet würde bzw. stets erfüllt wäre.

Die Zeile 31 zeigt die Übertragung zweier Datenpakete 24, 26 von der Datenquelle 3 in den Empfangspuffer 12. Das erste Datenpaket 24 wird hierbei zum Zeitpunkt 23 empfangen und das zweite Datenpaket 26 zum Zeitpunkt 25.

Zum Zeitpunkt 23 erkennt die Transfereinrichtung 15, dass die Sendeschnittstelle 19 zum Senden nutzbar ist, beispielsweise da eine Sendeantenne im Empfangsbereich einer Empfangsantenne der Empfangseinrichtung 6 ist, und dass der Sendepuffer 16 leer ist. Daher wird der Sendepuffer 16 ausgewählt, um die Daten des ersten Datenpakets 24 dort hin zu kopieren. Sobald Teile des Datenpakets 24 in dem Sendepuffer 16 einlaufen, wird durch die Sendeschnittstelle 19 mit dem Senden dieser Daten begonnen.

In der Zeile 32 in Fig. 2 ist der zeitliche Ablauf des Empfangs des ersten Datenpakets 24 durch die Empfangseinrichtung 6 visualisiert. Der Beginn des Empfangs ist um das Zeitintervall 27 von dem Zeitpunkt 23 beabstandet, da das Kopieren der Daten in den Empfangspuffer 16 und insbesondere die drahtlose Übertragung zu der Empfangseinrichtung 6 eine gewisse Zeit benötigt. Zudem ist durch Vergleich der Zeilen 31 und 32 gut erkennbar, dass zum Empfang des Datenpakets 24 durch die Empfangseinrichtung 6 ein längeres Zeitintervall 28 erforderlich ist, als das Zeitintervall zwischen den Zeitpunkten 23 und 25, da bei einer drahtlosen Übertragung in der Regel eine niedrigere Übertragungsrate erreicht wird.

Da zunächst ausgegangen wird, dass die Freigabebedingung 22 nicht geprüft wird, werden auch die Daten des zweiten Datenpakets 26 unmittelbar nach ihren Speichern im Empfangspuffer 12 in einen ausgewählte Sendepuffer 17 und von dort über die Sendeschnittstelle 20 zur Empfangseinrichtung 6 übertragen. Der zeitliche Verlauf des Empfangs des zweiten Datenpakets 26 durch die Empfangseinrichtung 6 für diesen Fall ist in der Zeile 33 der Fig. 2 visualisiert. Wie bereits obig zum ersten Datenpaket 24 erläutert ist auch der Empfang des zweiten Datenpakets 26 um ein kurzes Zeitintervall 29 verzögert, das für die Übertragung der Daten einerseits vom Empfangspuffer 12 zum Sendepuffer 17 und andererseits vom Sendepuffer 17 zur Empfangseinrichtung 6 erforderlich ist. Zudem ist das Zeitintervall 30 zum Empfangen des Datenpakets 26 gegenüber der in Zeile 31 gezeigten Dauer, die zum Schreiben des Datenpakets 26 in den Empfangspuffer 12 erforderlich ist, verlängert, da bei der drahtlosen Übertragung geringere Übertragungsraten als bei der vorangehenden kabelgebundenen Übertragung erreicht werden.

Die Übertragung der Datenpakete 24, 26 von der Empfangseinrichtung 6 zur Verarbeitungseinrichtung 7 soll erst dann erfolgen, wenn das jeweilige Paket 24, 26 vollständig durch die Empfangseinrichtung 6 empfangen wurde. Da typischerweise bei der drahtgebundenen Übertragung von der Empfangseinrichtung 6 zur Verarbeitungseinrichtung 7 höhere Übertragungsraten erreicht werden als für die drahtlose Übertragung, kann nur so eine unterbrechungsfreie Übertragung des Datenpakets erreicht werden.

Da jedoch der Empfang des zweiten Datenpakets 26 bereits zum Zeitpunkt 37 abgeschlossen ist, also vor dem Zeitpunkt 46, zu dem der Empfang des ersten Datenpakets 28 abgeschlossen ist, wird von der Empfangseinrichtung 6 zuerst das Datenpaket 26 übertragen und erst nach Abschluss dieser Übertragung das Datenpaket 24. Der zeitliche Ablauf der übertragenen Datenpakete 24, 26 von der Empfangseinrichtung 6 zu der Verarbeitungseinrichtung 7 ist in Zeile 34 in Fig. 2 dargestellt. Durch Vergleiche der Zeilen 31 und 34 ist offensichtlich, dass die Datenpakete 24, 26 durch die Verarbeitungseinrichtung 7 in umgekehrter Sendereihenfolge empfangen werden, wo mit in der Verarbeitungseinrichtung 7 vor einer weiteren Verarbeitung eine Umsortierung der Paketreihenfolge erforderlich wäre.

Wie obig erläutert, kann diese ungewünschte Änderung der Paketreihenfolge vermieden werden, indem eine Übertragung des zweiten Datenpakets 26 in den ausgewählten Sendepuffer 17 solange verzögert wird, bis eine den Füllgrad eines weiteren Sendepuffers, im Beispiel des Sendepuffers 16, auswertende Freigabebedingung erfüllt ist. Ein Beispiel für die Auswertung einer Freigabebedingung wird im Folgenden genauer mit Bezug auf Fig. 3 erläutert.

Fig. 3 zeigt schematisch zum einen die im Empfangspuffer 12 gespeicherten Datenpakete 24, 26 und zum anderen das bereits in den Sendepuffer übertragene Datenpaket 24. Da die Übertragung des Datenpakets 24 in den Sendepuffer 16 bereits abgeschlossen ist, müsste dieses im gezeigten Verfahrenszustand nicht länger im Empfangspuffer 12 gespeichert sein. FIFO-Puffer sind jedoch häufig als Ringpuffer ausgestaltet, wobei bereits ausgelesene Daten bereits von einem Lesezeiger überstrichen wurden, jedoch im Speicher bleiben. Die Daten werden erste dann überschrieben, wenn ein Schreibzeiger die zu überschreibenden Daten erreicht.

Zum Zeitpunkt 25, zu dem das zweite Datenpaket empfangen wird, ist das erste Datenpaket bereits im Wesentlichen vollständig in den Sendepuffer 16 übertragen, so dass ein Schreibzeiger 41 dieses Puffers auf das Ende des Datenpakets 24 zeigt. Da zudem, wie insbesondere in Zeile 32 in der Fig. 2 zu erkennen ist, bereits ungefähr die Hälfte der Daten des Datenpakets 24 an die Empfangseinrichtung 6 gesendet wurde und somit bereits aus dem Sendepuffer 16 ausgelesen wurde, zeigt der Lesezeiger 40 des Sendepuffers 16 ungefähr auf die Mitte des zweiten Datenpakets 24. Der Abstand zwischen Lese- und Schreibzeiger 40, 41 entspricht der Länge 43 des verbleibenden Teildatenpakets 42 des Datenpakets 24, das noch nicht über die dem Sendepuffer 16 zugeordnete Sendeschnittstelle 19 an die Empfangseinrichtung 6 gesendet wurde. Die Länge 43 kann auch als Füllgrad 39 des Sendepuffers 16 bezeichnet werden.

Wie in Fig. 3 deutlich zu erkennen ist, ist die Länge 43 größer als die Länge 38 des Datenpakets 26. Dies führt jedoch dazu, dass das Datenpaket 26, wenn seine Daten unmittelbar in den Sendepuffer 17 übertragen würden, voraussichtlich vor dem Datenpaket 24 vollständig zur Empfangseinrichtung 6 übertragen würde, was zur obig erläuterten Änderung der Paketreihenfolge führen würde. Zum Zeitpunkt 25 soll daher die Freigabebedingung nicht erfüllt sein.

Fig. 3 zeigt zudem den Lesezeiger 40' an einer Position, die er zum Zeitpunkt 62, also nach Ende des Zeitintervalls 45, in Zeile 35 der Fig. 2 einnimmt. Aufgrund des kontinuierlichen Sendens und somit des Auslesens von Daten des Datenpakets 24 hat sich der Lesezeiger hierbei nach rechts zum Schreibzeiger 41 hin verschoben, womit die Länge 43' beziehungsweise der Füllgrad 39' des nun verbleibenden Teildatenpakets 42' kleiner als die Länge 38 des Datenpakets 26 ist.

Würde davon ausgegangen, dass bei der Übertragung der Datenpakete 24, 26 stets die gleichen Übertragungsraten und Latenzen resultieren, könnte in diesem Fall die Freigabebedingung stets erfüllt sein und somit die Übertragung des zweiten Datensatzes 26 in den Sendepuffer 17 beginnen. Um eine korrekte Paketreihenfolge jedoch auch in Fällen robust sicherstellen zu können, in denen Übertragungszeiten beziehungsweise Raten, beispielsweise aufgrund von verschiedenen möglichen Übertragungspfaden, möglichen Störungen des drahtlosen Übertragungspfades etc., variieren, ist im gezeigten Beispiel die Freigabebedingung nur dann erfüllt, wenn die Summe aus der Länge 43, 43' des verbleibenden Teildatenpakets 42, 42' und einem Offsetwert 44 kleiner ist als die Länge 38 des Datenpakets 26.

Durch Nutzung dieser Freigabebedingung wird die Übertragung des zweiten Datenpakets 26 um das Zeitintervall 45 verzögert, wie aus einem Vergleich der Zeilen 33, 35 in Fig. 2 erkennbar ist. Dies führt dazu, dass der Empfang des Datenpakets 26 durch die Empfangseinrichtung 6 erst zu einem Zeitpunkt abgeschlossen ist, der nach dem Zeitpunkt 46 liegt, zu dem das erste Datenpaket 24 vollständig empfangen ist. Dies führt dazu, dass bei Nutzung der Freigabebedingung 22 die Datenpakete 24, 26 in der korrekten Reihenfolge von der Empfangseinrichtung 6 zur Verarbeitungseinrichtung 7 übertragen werden, wie in Zeile 36 in Fig. 2 dargestellt ist.

Fig. 4 zeigt schematisch ein konkretes Beispiel einer medizinischen Bildgebungseinrichtung 2, nämlich eines Computertomographen, in der die obig erläuterte Lehre zur Datenübertragung genutzt wird. Die Bildgebungseinrichtung 2 umfasst eine Basis 47 und ein bezüglich der Basis 47 drehbar gelagertes Gestell 46, nämlich eine Gantry, die eine Röntgenquelle 50 und als Sensoreinrichtungen 48, 49, die über die Empfangseinrichtung 55 Daten an eine Verarbeitungseinrichtung 61 bereitstellen sollen, einen bildgebenden Röntgendetektor und eine Spannungsüberwachung der Röntgenröhre 50 trägt. Die Übertragung der Messdaten der als Datenquelle 3, 4 fungierenden Sensoreinrichtungen 48, 49 erfolgt über eine nur schematisch dargestellte Datenübertragungseinrichtung 63, die abgesehen von einer anderen Kanalzahl jedoch im Wesentlichen der in Fig. 1 dargestellten Datenübertragungseinrichtung 8 entspricht. Die großflächigen Sendeantennen 51, 52, 53, 54 sind hierbei jeweils Teil einer der Sendeschnittstelle der Datenübertragungseinrichtung 63 und somit, wie bereits obig erläutert, durch einen jeweiligen Sendepuffer beschickt.

Die basisseitige Empfangseinrichtung 55 verfügt über fünf Empfangskanäle, denen jeweils eine der Empfangsantennen 56, 57, 58, 59, 60 zugeordnet ist. Über diese Empfangsantennen parallel empfangenen Datenströme werden anschließend serialisiert und zur Verarbeitungseinrichtung 61 übertragen. Durch die obig erläuterte Auswertung einer Freigabebedingung durch die Datenübertragungseinrichtung 63 bzw. deren Transfereinrichtung kann, wie erläutert, sichergestellt werden, dass trotz dieser abschnittsweisen Nutzung von parallelen Übertragungspfaden robust die korrekte Paketreihenfolge erhalten bleibt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Datenübertragungseinrichtung zur Übertragung von Datenpaketen (24, 26), umfassend
- wenigstens eine Empfangsschnittstelle (-9 - 11) zum Empfangen von Datenpaketen (24, 25) von einer jeweiligen Datenquelle (3 - 5),
- einen jeweiligen Empfangspuffer (12 - 14) zur Zwischenspeicherung der über die jeweilige Empfangsschnittstelle (9 - 11) empfangenen Datenpakete (24, 25),
- eine Transfereinrichtung (15) zur Übertragung der Datenpakete (24, 25) von dem jeweiligen Empfangspuffer (12 - 14) in einen für das jeweilige Datenpaket (24, 25) aus mehreren vorhandenen Sendepuffern (16 - 18) ausgewählten Sendepuffer (16 - 18), und
- eine jeweilige Sendeschnittstelle (19 - 21) zum Senden der in dem jeweiligen Sendepuffer (16 - 18) gespeicherten Datenpakete (24, 25) an eine Empfangseinrichtung (6, 55),
wobei die Transfereinrichtung (15) dazu eingerichtet ist, die Übertragung des jeweiligen Datenpakets (24, 25) von dem jeweiligen Empfangspuffer (12 - 14) in den ausgewählten Sendepuffer (16 - 18) erst dann durchzuführen, wenn eine Freigabebedingung (22) erfüllt ist, deren Erfüllung von einem Füllgrad (39, 39') eines von dem ausgewählten Sendepuffer (16 - 18) unterschiedlichen weiteren der Sendepuffer (16 - 18) abhängt,
**dadurch gekennzeichnet, dass** der Füllgrad (39, 39') eine Länge (43, 43') eines noch nicht über die dem weiteren Sendepuffer (16 - 18) zugeordnete Sendeschnittstelle (19 - 21) gesendeten, verbleibenden Teildatenpakets (42, 42') eines vorangehend in den weiteren Sendepuffer (16 - 18) übertragenen weiteren Datenpakets (24, 26) beschreibt, wobei die Freigabebedingung (22) erfüllt wird, wenn die Länge (43, 43') des in den ausgewählten Sendepuffer (16 - 18) zu übertragenden Datenpakets (24, 26) die Länge des Teildatenpakets (42, 42') oder die Summe aus der Länge (43, 43') des Teildatenpakets (42, 42') und einem vorgegebenen Offsetwert (44) erreicht oder überschreitet.

2. Datenübertragungseinrichtung nach Anspruch 1, **dadurch gekennzeichnet, dass** die Transfereinrichtung (15) dazu eingerichtet ist, im Rahmen der Auswertung der Freigabebedingung (22) jenen der Sendepuffer (16 - 18) als weiteren Sendepuffer (16 - 18) zu berücksichtigen, in den ein weiteres Datenpaket (24, 26) übertragen wurde, das unmittelbar vor dem in den ausgewählten Sendepuffer (16 - 18) zu übertragenden Datenpaket (24, 26) empfangen wurde.

3. Datenübertragungseinrichtung nach Anspruch 1, **dadurch gekennzeichnet, dass** die Transfereinrichtung (15) dazu eingerichtet ist, im Rahmen der Auswertung der Freigabebedingung (22) jenen der Sendepuffer (16 - 18) als weiteren Sendepuffer (16 - 18) zu berücksichtigen, in den ein weiteres Datenpaket (24, 26) übertragen wurde, das unmittelbar vor dem in den ausgewählten Sendepuffer (16 - 18) zu übertragenden Datenpaket (24, 26) über die gleiche Empfangsschnittstelle (9 - 11) empfangen wurde.

4. Datenübertragungseinrichtung nach einem der vorangehenden Ansprüche, **dadurch gekennzeichnet, dass** die Empfangsschnittstelle (9 - 11) drahtgebunden mit der jeweiligen Datenquelle (3 - 5) verbunden oder verbindbar ist und/oder dass die jeweilige Sendeschnittstelle (19 -21) zur drahtlosen Übertragung des jeweiligen Datenpakets (24, 26) an die Empfangseinrichtung (6, 55) ausgebildet ist.

5. Datenübertragungseinrichtung nach einem der vorangehenden Ansprüche, **dadurch gekennzeichnet, dass** die Datenübertragungseinrichtung (8, 63) zur Übertragung von Messdaten einer medizinischen Bildgebungseinrichtung (1, 2) als Datenpakete (24, 26) dient.

6. Medizinische Bildgebungseinrichtung, **dadurch gekennzeichnet, dass** die Bildgebungseinrichtung (1, 2) eine Datenübertragungseinrichtung (8, 63) nach einem der vorangehenden Ansprüche umfasst.

7. Medizinische Bildgebungseinrichtung nach Anspruch 6, **dadurch gekennzeichnet, dass** sie eine Sensoreinrichtung (48, 49) umfasst, wobei die Sensoreinrichtung (48, 49) dazu eingerichtet ist, als die Datenquelle (3 - 5) oder über eine als Datenquelle (3 - 5) dienende Verarbeitungseinrichtung Datenpakete (24, 26) an die Datenübertragungseinrichtung (8, 63) bereitzustellen.

8. Medizinische Bildgebungseinrichtung nach Anspruch 6 oder 7, **dadurch gekennzeichnet, dass** sie ein bezüglich einer Basis (47) bewegliches gelagertes Gestell (46) umfasst, das die Datenquelle (3, 4, 5) und die Datenübertragungseinrichtung (8, 63) umfasst, wobei die Empfangseinrichtung (6, 55) ortsfest bezüglich der Basis (47) angeordnet ist.

9. Medizinische Bildgebungseinrichtung nach einem der Ansprüche 6 bis 8, **dadurch gekennzeichnet, dass** die Bildgebungseinrichtung (1, 2) ein Computertomograph ist oder einen Computertomograph umfasst, und/oder dass die Sensoreinrichtung (48, 49) ein Röntgensensor ist oder einen Röntgensensor umfasst, und/oder dass das beweglich gelagerte Gestell (46) eine drehbar gelagerte Gantry ist.

10. Verfahren zur Übertragung von Datenpaketen (24, 26), wobei
- Datenpakete (24, 26) von einer jeweiligen Datenquelle (3 - 5) empfangen und in einem Empfangspuffer (12 - 14) zwischengespeichert werden,
- die Datenpakete (24, 26) von dem Empfangspuffer (12 - 14) in einen für das jeweilige Datenpaket (24, 26) aus mehreren vorhandenen Sendepuffern (16 - 18) ausgewählten Sendepuffer (16 - 18) übertragen werden, wobei die Übertragung des jeweiligen Datenpakets (24, 26) von dem jeweiligen Empfangspuffer (12 - 15) in den ausgewählten Sendepuffer (16 - 18) erst bei Erfüllung einer Freigabebedingung (22) erfolgt, deren Erfüllung von einem Füllgrad (39, 39') eines von dem ausgewählten Sendepuffer (16 - 18) unterschiedlichen weiteren der Sendepuffer (16 - 18) abhängt, und
- die in dem jeweiligen Sendepuffer (16 - 18) gespeicherten Datenpakete (24, 26) durch eine jeweilige dem Sendepuffer (16 - 18) zugeordnete Sendeschnittstelle (19 - 21) an eine Empfangseinrichtung (6, 55) gesendet werden,
**dadurch gekennzeichnet, dass** der Füllgrad (39, 39') eine Länge (43, 43') eines noch nicht über die dem weiteren Sendepuffer (16 - 18) zugeordnete Sendeschnittstelle (19 - 21) gesendeten, verbleibenden Teildatenpakets (42, 42') eines vorangehend in den weiteren Sendepuffer (16 - 18) übertragenen weiteren Datenpakets (24, 26) beschreibt, wobei die Freigabebedingung (22) erfüllt wird, wenn die Länge (43, 43') des in den ausgewählten Sendepuffer (16 - 18) zu übertragenden Datenpakets (24, 26) die Länge des Teildatenpakets (42, 42') oder die Summe aus der Länge (43, 43') des Teildatenpakets (42, 42') und einem vorgegebenen Offsetwert (44) erreicht oder überschreitet.

11. Verfahren nach Anspruch 10, **dadurch gekennzeichnet, dass** durch das Verfahren als Datenpakete (24, 26) Messdaten einer medizinischen Bildgebungseinrichtung (1, 2) übertragen werden, und/oder dass die Datenpakete (24, 26) von der jeweiligen Datenquelle (3 - 5) über eine drahtgebundene Verbindung empfangen werden, und/oder dass die in dem jeweiligen Sendepuffer (16 - 18) gespeicherten Datenpakete (24, 26) durch die jeweilige dem Sendepuffer (16 - 18) zugeordnete Sendeschnittstelle (19 - 21) über eine drahtlose Verbindung an die Empfangseinrichtung (6, 55) gesendet werden.

12. Computerprogramm **dadurch gekennzeichnet, dass** es Anweisungen umfasst, bei deren Ausführung durch eine Transfereinrichtung (15) Datenpakete (24, 26) von einem Empfangspuffer (12 - 14) in einen für das jeweilige Datenpaket (24, 26) aus mehreren vorhandenen Sendepuffern (16 - 18) ausgewählten Sendepuffer (16 - 18) übertragen werden, wobei die Übertragung des jeweiligen Datenpakets (24, 26) von dem jeweiligen Empfangspuffer (12 - 15) in den ausgewählten Sendepuffer (16 - 18) erst bei Erfüllung einer Freigabebedingung (22) erfolgt, deren Erfüllung von einem Füllgrad (39, 39') eines von dem ausgewählten Sendepuffer (16 - 18) unterschiedlichen weiteren der Sendepuffer (16 - 18) abhängt, **dadurch gekennzeichnet, dass** der Füllgrad (39, 39') eine Länge (43, 43') eines noch nicht über die dem weiteren Sendepuffer (16 - 18) zugeordnete Sendeschnittstelle (19 - 21) gesendeten, verbleibenden Teildatenpakets (42, 42') eines vorangehend in den weiteren Sendepuffer (16 - 18) übertragenen weiteren Datenpakets (24, 26) beschreibt, wobei die Freigabebedingung (22) erfüllt wird, wenn die Länge (43, 43') des in den ausgewählten Sendepuffer (16 - 18) zu übertragenden Datenpakets (24, 26) die Länge des Teildatenpakets (42, 42') oder die Summe aus der Länge (43, 43') des Teildatenpakets (42, 42') und einem vorgegebenen Offsetwert (44) erreicht oder überschreitet.

13. Computerlesbarer Datenträger, **dadurch gekennzeichnet, dass** er ein Computerprogramm nach Anspruch 12 umfasst.

## Claims

1. Data transmission device for transmitting data packets (24, 26), comprising
- at least one receive interface (3 - 5) for receiving data packets (24, 25) from a respective data source (2 - 5),
- a respective receive buffer (12 - 14) for buffering the data packets (24, 25) received via the respective receive interface (2 - 5),
- a transfer device (15) for transferring the data packets (24, 25) from the respective receive buffer (12 - 14) to a transmit buffer (16 - 18) selected for the respective data packet (24, 25) from a plurality of existing transmit buffers (16 - 18), and
- a respective transmit interface (19 - 21) for transmitting the data packets (24, 25) stored in the respective transmit buffer (16 - 18) to a receiving device (6, 55),
wherein the transfer device (15) is designed to transfer the respective data packet (24, 25) from the respective receive buffer (12 - 14) into the selected transmit buffer (16 - 18) only when an enable condition (22) is fulfilled, the fulfilment of which depends on a fill level (39, 39') of one of the transmit buffers (16 - 18) other than the selected transmit buffer (16 - 18),
**characterised in that** the fill level (39, 39') describes a length (43, 43') of a remaining partial data packet (42, 42') of another data packet (24, 26) previously transferred into the other transmit buffer (16 - 18), which partial data packet has not yet been transmitted via the transmit interface (19 - 21) assigned to the other transmit buffer (16 - 18), wherein the enable condition (22) is fulfilled if the length (43, 43') of the data packet (24, 26) to be transferred to the selected transmit buffer (16 - 18) attains or exceeds the length of the partial data packet (42, 42') or the sum of the length (43, 43') of the partial data packet (42, 42') and a predetermined offset value (44).

2. Data transmission device according to claim 1, **characterised in that**, for evaluating the enable condition (22), the transfer device (15) is designed to consider, as another transmit buffer (16 - 18), the transmit buffer (16 - 18) into which another data packet (24, 26) has been transferred which was received immediately prior to the data packet (24, 26) to be transferred into the selected transmit buffer (16 - 18).

3. Data transmission device according to claim 1, **characterised in that**, for evaluating the enable condition (22), the transfer device (15) is designed to consider, as another transmit buffer (16 - 18), the transmit buffer (16 - 18) into which another data packet (24, 26) has been transferred which was received via the same receive interface (9 - 11) immediately before the data packet (24, 26) to be transferred to the selected transmit buffer (16 - 18) .

4. Data transmission device according to one of the preceding claims, **characterised in that** the receive interface (9 - 11) is connected or connectable by wire to the respective data source (3 - 5) and/or that the respective transmit interface (19 - 21) is designed for wireless transmission of the respective data packet (24, 26) to the receiving device (6, 55).

5. Data transmission device according to one of the preceding claims, **characterised in that** the data transmission device (8, 63) is used for transmitting measurement data of a medical imaging device (1, 2) as data packets (24, 26).

6. Medical imaging device, **characterised in that** the imaging device (1, 2) comprises a data transmission device (8, 63) according to one of the preceding claims.

7. Medical imaging device according to claim 6, **characterised in that** it comprises a sensor device (48, 49), wherein the sensor device (48, 49) is designed to supply data packets (24, 26) to the data transmission device (8, 63) as the data source (3 - 5) or via a processing device used as the data source (3 - 5).

8. Medical imaging device according to claim 6 or 7, **characterised in that** it comprises a framework (46) movably mounted with respect to a base (47) and incorporating the data source (3, 4, 5) and the data transmission device (8, 63), wherein the receiving device (6, 55) is fixed in relation to the base (47).

9. Medical imaging device according to one of claims 6 to 8, **characterised in that** the imaging device (1, 2) is a CT scanner or comprises a CT scanner, and/or that the sensor device (48, 49) is an X-ray sensor or comprises an X-ray sensor, and/or that the movably mounted framework (46) is a rotatable gantry.

10. Method for transmitting data packets (24, 26), wherein
- data packets (24, 26) are received from a respective data source (3 - 5) and are temporarily stored in a receive buffer (12 - 14),
- the data packets (24, 26) are transferred from the receive buffer (12 - 14) into a transmit buffer (16 - 18) selected for the respective data packet (24, 26) from a plurality of existing transmit buffers (16 - 18), wherein the respective data packet (24, 26) is only transferred from the respective receive buffer (12 - 15) into the selected transmit buffer (16 - 18) if an enable condition (22) is fulfilled, the fulfilment of which depends on a fill level (39, 39') of one of the transmit buffers (16 - 18) other than the selected transmit buffer (16 - 18), and
- the data packets (24, 26) stored in the respective transmit buffer (16 - 18) are transmitted to a receiving device (6, 55) by a respective transmit interface (19 - 21) assigned to the transmit buffer (16 - 18)
**characterised in that** the fill level (39, 39') describes a length (43, 43') of a remaining partial data packet (42, 42') of another data packet (24, 26) previously transferred into the other transmit buffer (16 - 18), which partial data packet has not yet been transmitted via the transmit interface (19 - 21) assigned to the other transmit buffer (16 - 18), wherein the enable condition (22) is fulfilled if the length (43, 43') of the data packet (24, 26) to be transferred to the selected transmit buffer (16 - 18) attains or exceeds the length of the partial data packet (42, 42') or the sum of the length (43, 43') of the partial data packet (42, 42') and a predetermined offset value (44) .

11. Method according to claim 10, **characterised in that** measurement data of a medical imaging device (1, 2) is transmitted by the method as data packets (24, 26), and/or that the data packets (24, 26) are received from the respective data source (3 - 5) via a wired connection and/or **in that** the data packets (24, 26) stored in the respective transmit buffer (16 - 18) are transmitted to the receiving device (6, 55) via a wireless connection by the respective transmit interface (19 - 21) assigned to the transmit buffer (16 - 18).

12. Computer program, **characterised in that** it comprises instructions, during the execution of which data packets (24, 26) are transferred by a transfer device (15) from a receive buffer (12 - 14) to a transmit buffer (16 - 18) selected for the respective data packet (24, 26) from a plurality of existing transmit buffers (16 - 18,) wherein transfer of the respective data packet (24, 26) from the respective receive buffer (12 - 15) into the selected transmit buffer (16 - 18) takes place only if an enable condition is fulfilled (22), the fulfilment of which depends on a fill level (39, 39') of one of the transmit buffers (16 - 18) other than the selected transmit buffer (16 - 18), **characterised in that** the fill level (39, 39') describes a length (43, 43') of a remaining partial data packet (42, 42') of another data packet (24, 26) previously transferred into the other transmit buffer (16 - 18), which partial data packet has not yet been transmitted via the transmit interface (19 - 21) assigned to the other transmit buffer (16 - 18), wherein the enable condition (22) is fulfilled if the length (43, 43') of the data packet (24, 26) to be transferred to the selected transmit buffer (16 - 18) attains or exceeds the length of the partial data packet (42, 42') or the sum of the length (43, 43') of the partial data packet (42, 42') and a predetermined offset value (44).

13. Computer-readable data carrier, **characterised in that** it comprises a computer program according to claim 12.

## Revendications

1. Dispositif de transmission de données pour la transmission de paquets (24, 26) de données, comprenant
- au moins une interface (-9 - 11) de réception pour la réception de paquets (24, 25) de données d'une source (3 - 5) de données respective,
- un tampon (12 - 14) de réception respectif pour la mise en mémoire tampon des paquets (24, 25) de données reçus par les interfaces (9 - 11) de réception respectives,
- un dispositif (15) de transfert pour la transmission des paquets (24, 25) de données du tampon (12 - 14) de réception respectif à un tampon (16 - 18) d'émission du paquet (24, 25) de données respectif sélectionné parmi plusieurs tampons (16 - 18) d'émission présents, et
- une interface (19 - 21) d'émission respective pour l'envoi des paquets (24, 25) de données mis en mémoire dans le tampon (16 - 18) d'émission respectif à un dispositif (6, 55) de réception,
dans lequel le dispositif (15) de transfert est agencé pour n'effectuer la transmission du paquet (24, 25) de données respectif du tampon (12 - 14) de réception respectif au tampon (16 - 18) d'émission sélectionné, que si une condition (22) de validation est satisfaite, dont la satisfaction dépend d'un degré (39, 39') de remplissage d'un autre des tampons (16 - 18) d'émission différent du tampon (16 - 18) d'émission sélectionné,
**caractérisé en ce que** le degré (39, 39') de remplissage décrit une longueur (43, 43') d'un paquet (42, 42') de données partiel, restant, pas encore envoyé par l'interface (19 - 21) d'émission, associée à l'autre tampon (16 - 18) d'émission, d'un autre paquet (24, 26) de données transmis précédemment à l'autre tampon (16 - 18) d'émission, dans lequel la condition (22) de validation est satisfaite si la longueur (43, 43') du paquet (24, 26) de données à transmettre à l'autre tampon (16 - 18) d'émission sélectionné atteint ou dépasse la longueur du paquet (42, 42') de données partiel ou la somme composée de la longueur (43, 43') du paquet (42, 42') de données partiel et d'une valeur (44) de décalage donnée à l'avance.

2. Dispositif de transmission de données suivant la revendication 1, **caractérisé en ce que** le dispositif (15) de transfert est agencé pour prendre en compte, comme autre tampon (16 - 18) d'émission, dans le cadre de l'évaluation de la condition (22) de validation, celui des tampons (16 - 18) d'émission, dans lequel a été transmis un paquet (24, 26) de données, autre que celui qui a été reçu juste avant le paquet (24, 26) de données à transmettre au tampon (16 - 18) d'émission sélectionné.

3. Dispositif de transmission de données suivant la revendication 1, **caractérisé en ce que** le dispositif (15) de transfert est agencé pour tenir compte, dans le cadre de l'évaluation de la condition (22) de validation, celui des tampon (16 - 18) d'émission comme autre tampon (16 - 18) d'émission, auquel un autre paquet (24, 26) de données a été transmis, qui a été reçu par la même interface (9 - 11) de réception juste avant le paquet (24, 26) de données à transmettre au tampon (16 - 18) d'émission sélectionné.

4. Dispositif de transmission de données suivant l'une des revendications précédentes, **caractérisé en ce que** l'interface (9 - 11) de réception est reliée sans fil à la source (3 - 5) de données respective ou peut l'être et/ou **en ce que** l'interface (19 - 21) d'émission respective est constituée pour la transmission sans fil du paquet (24, 26) de données respectif au dispositif (6, 55) de réception.

5. Dispositif de transmission de données suivant l'une des revendications précédentes, **caractérisé en ce que** le dispositif (8, 63) de transmission de données sert à la transmission comme paquets (24, 26) de données de données de mesure d'un dispositif (1, 2) d'imagerie médicale.

6. Dispositif d'imagerie médicale, **caractérisé en ce que** le dispositif (1, 2) d'imagerie médicale comprend un dispositif (8, 63) de transmission de données suivant l'une des revendications précédentes.

7. Dispositif d'imagerie médicale suivant la revendication 6, **caractérisé en ce qu'**il comprend un dispositif (48, 49) capteur, dans lequel le dispositif (48, 49) capteur est agencé pour préparer, sur le dispositif (8, 63) de transmission de données, des paquets (24, 26) de données comme source (3 - 5) de données ou par un dispositif de traitement servant de source (3 - 5) de données.

8. Dispositif d'imagerie médicale suivant la revendication 6 ou 7, **caractérisé en ce qu'**il comprend un bâti (46) monté mobile par rapport à une base (47), qui comprend la source (3, 4, 5) de données et le dispositif (8, 63) de transmission de données, dans lequel le dispositif (6, 55) de réception est monté fixe en position par rapport à la base (47).

9. Dispositif d'imagerie médicale suivant l'une des revendications 6 à 8, **caractérisé en ce que** le dispositif (1, 2) d'imagerie médicale est un tomodensitomètre assisté par ordinateur ou comprend un tomodensitomètre assisté par ordinateur, et/ou **en ce que** le dispositif (48, 49) capteur est un capteur de rayons X ou comprend un capteur de rayons X, ou **en ce que** le bâti (46) monté mobile est un portique monté tournant.

10. Procédé de transmission de paquets (24, 26) de données, dans lequel
- on reçoit des paquets (24, 26) de données d'une source (3 - 5) de données respective et on les met en mémoire tampon dans une mémoire (12 - 14) tampon,
- on transmet les paquets (24, 26) de données de la mémoire (12 - 14) de réception à un tampon (16 - 18) d'émission pour le paquet (24, 26) de données respectif sélectionné parmi plusieurs mémoires (16 - 18) d'émission présentes, dans lequel la transmission du paquet (24, 26) de données respectif du tampon (12 - 15) de réception respectif aux tampons (16 - 18) d'émission sélectionnés n'a lieu que si une condition (22) de validation est satisfaite, dont la satisfaction dépend d'un degré (39, 39') de remplissage d'un autre des tampons (16 - 18) d'émission différent du tampon (16 - 18) d'émission sélectionné, et
- on envoie à un dispositif (6, 55) de réception, par une interface (19 - 21) d'émission respective associée aux tampons (16 - 18) d'émission, les paquets (24, 26) de données mis en mémoire dans le tampon (16 - 18) d'émission respectif,
**caractérisé en ce que** le degré (39, 39') de remplissage décrit une longueur (43, 43') d'un paquet (42, 42') de données partiel restant, pas encore envoyé par l'interface (19 - 21) d'émission associée à l'autre tampon (16 - 18) d'émission, d'un autre paquet (24, 26) de données transmis précédemment à l'autre tampon (16 - 18) d'émission, dans lequel la condition (22) de validation est satisfaite si la longueur (43, 43') du paquet (24, 26) de données à transmettre à l'autre tampon (16 - 18) d'émission sélectionné atteint ou dépasse la longueur du paquet (42, 42') de données partiel ou la somme composée de la longueur (43, 43') du paquet (42, 42') de données partiel et d'une valeur (44) de décalage donnée à l'avance.

11. Procédé suivant la revendication 10, **caractérisé en ce que**, pour le procédé, on transmet comme paquets (24, 26) de données des données de mesure d'un dispositif (1, 2) d'imagerie médicale, et/ou on reçoit les paquets (24, 26) de la source (3 - 5) de données respective par une liaison par fil, et/ou **en ce que** on envoie au dispositif (6, 55) de réception, par une liaison sans fil par l'interface (19 - 21) d'émission associée au tampon (16 - 18) d'émission respectif, les paquets (24, 26) de données mis en mémoire dans le tampon (16 - 18) d'émission respectif.

12. Programme d'ordinateur, **caractérisé en ce qu'**il comprend des instructions, dont la réalisation par un dispositif (15) de transfert font que des paquets (24, 26) de données sont transmis d'un tampon (12 - 14) de réception à un tampon (16 - 18) d'émission sélectionné pour le paquet (24, 26) de données respectif sélectionné parmi plusieurs tampons (16 - 18) d'émission présents, dans lequel la transmission du paquet (24, 26) de données respectif du tampon (12 - 15) de réception respectif au tampon (16 - 18) d'émission sélectionné, n'a lieu que si une condition (22) de validation est satisfaite, dont la validation dépend d'un degré (39, 39') de remplissage d'un autre des tampons (16, 18) d'émission différents du tampon (16 - 18) d'émission sélectionné, **caractérisé en ce que** le degré (39, 39') de remplissage décrit une longueur (43, 43') d'un paquet (42, 42') de données partiel restant, pas encore envoyé par l'interface (19 - 21) d'émission associée à l'autre tampon (16 - 18) d'émission, d'un autre paquet (24, 26) de données transmis précédemment à l'autre tampon (16 - 18) d'émission, dans lequel la condition (22) de validation est satisfaite si la longueur (43, 43') du paquet (24, 26) de données à transmettre à l'autre tampon (16 - 18) d'émission sélectionné atteint ou dépasse la longueur du paquet (42, 42') de données partiel ou la somme composée de la longueur (43, 43') du paquet (42, 42') de données partiel et d'une valeur (44) de décalage donnée à l'avance.

13. Support de données, déchiffrable par ordinateur, **caractérisé en ce qu'**il comprend un programme d'ordinateur suivant la revendication 12.
